# EUROPEAN PATENT APPLICATION

(11) **EP 3 384 921 A1**
(43) Date of publication of application: **10.10.2018**
(21) Application number: 16869940.3
(22) Date of filing: 28.11.2016
(51) Int. Cl.: A61K 38/12, A61P 31/00, A61K 47/00, A61K 9/00

(54) **NEW USE OF THIOPEPTIN**

(30) Priority: 02.12.2015 CN 201510870586; 08.12.2015 CN 201510896693
(71) Applicant: Nanjing Biotica Pharmaceutical Company, Jiangsu 211198 (CN)
(72) Inventor: CHEN, Yijun, Nanjing Jiangsu 211198 (CN); QIAN, Junjian, Nanjing Jiangsu 211198 (CN); WU, Xuri, Nanjing Jiangsu 211198 (CN)
(74) Representative: Gong, Jinping
(86) International application number: PCT/CN2016/107404
(87) International publication number: WO 2017/092630

(57) **Abstract**

Provided in the present invention are a new use of thiopeptin in the preparation of drugs for treating diseases caused by clostridium difficile infections and complications thereof, and pharmaceutical preparations containing the thiopeptin for treating diseases caused by clostridium difficile infections and complications thereof.

## Description

### Field of invention

The present invention belongs to the field of a novel antibiotic and its application, particularly relates to thiopeptcin against *Clostridium difficile* and the use of it to overcome drug-resistance, as well as the formulations of thiopeptcin for oral administration.

### Technical background

*Clostridium difficile* is a gram-positive anaerobic bacillus, parasitizing approximately 3% of the general population in the intestine. *C. difficile* is a conditional pathogen, being regulated by other bacteria in human body and does not harm human health under normal circumstances. *C. difficile* infection (CDI) is usually caused by dysbacteriosis of the intestinal flora as a result of excessive or unreasonable use of broad-spectrum antibiotics (such as clindamycin, ampicillin, cephalosporins and lincomycin), and the incidence of CDI is rapidly increasing with the emergence of drug resistance. Studies have found that the main reason for CDI is the cytotoxic effects caused by endotoxins A and B from *C. difficile* (Expert Rev. Anti-infect. Ther. 2012, 10: 1405; Am. J. Med. Sci, 2010, 340: 247). CDI includes not only common pseudomembranous colitis and antibiotic-associated diarrhea, but also pyelonephritis, meningitis, abdominal pain, vaginal infections, bacteremia and gangrene. The clinical symptoms of these diseases vary from each other: some can be asymptomatic carriers, diarrhea, congestion, edema, erosion or explosive colitis; others can be sepsis or even shock death in severe cases.

Since *C. difficile* was confirmed as a cause of antibiotic-associated diarrhea in 1978, the morbidity and severity of CDI have increased year by year, especially the morbidity of CDI has increased 4-fold since the outbreak of high-toxic CDI in Quebec, Canada 15 years ago. According to the Hospital Infection Monitoring Center in Canada, the worldwide morbidity of CDI in adult is approximately 4.6/1,000 patients admitted, and the mortality rate is approximately 2.6/100 CDI patients (Expert Rev. Gastroenterol. Hepatol. 2010, 4: 409). In recent years, CDI in China has also shown a growing trend. According to the survey by Huangshan Hospital in Shanghai, the morbidity of acquired CDI in hospitals between 2007 and 2009 was approximately 13-17.1/10,000 patients admitted. (Clin. Infect. Dis. 2008, 47: 1606). Currently, metronidazole and vancomycin are the first-line drugs for combating and treating CDI (Am. J. Gastroenterol. 2013, 108: 478). Metronidazole is used to treat patients with mild or moderate infections for the first time, whereas oral vancomycin can be used to treat severe or relapsed patients. However, the recurrence rate of the treatment is as high as 15% to 35%, and the sensitivity-reduced or drug-resistant strains of these two therapeutic agents have been increasingly identified (Clin. Infect. Dis. 2012, 55: 71). In order to achieve a better therapeutic effect, fiddamycin, a new type of antibiotic for CDI was approved by the FDA in 2011, whose therapeutic effect is similar to vancomycin with a low recurrence rate. Unfortunately, the recurrence rate of highly virulent *C. difficile* strain NAPI/BI/027 is still as high as 24% with this newer drug (New Engl. J. Med. 2011, 364: 422), and sensitivity-reduced strains have also emerged clinically at the same time. Although pharmaceutical companies are developing new drugs for CDI treatments, none have reached to the market so far, such as Cadazolid, an experimental drug developed by Swiss company Actelion. Therefore, the increasing threat of recurrence during CDI treatments and the emergence of drug-resistant *C. difficile,* as well as the demand for more effective treatment of CDI, have positioned the urgent need for developing new types of first-line CDI drugs with novel structure and mechanism of action, exceptional activity, low recurrence rate as well as low drug resistance.

Thiopeptcin (also known as nocathiacin) is a thiopeptide-type secondary metabolite produced by *Amycolatopsis sp.* or *Nocardia sp.,* whose structure contains thiazole rings, a pyridine ring, an indole ring, a dehydroalanine, a dimethyl-aminoglucose and a fused tricyclic ring, is one of the most clinically promising candidates among thiopeptide antibiotics (J Antibiot 2003, 26:232). Thiopeptcin binds 23S rRNA in the large 50S subunit of bacterial ribosome and L11 protein, which in turn influences the elongation of peptide chains during protein synthesis to achieve the antibacterial effects. From *in vitro* studies, thiopeptcin has shown antibacterial effects against gram-positive aerobic bacteria at very low concentrations (ng/mL). However, practical applications of this compound on the treatments of anaerobic bacteria, especially *C. difficile,* remain elusive.

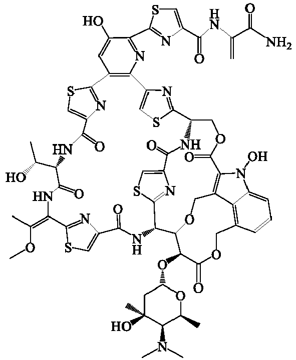

### Thiopeptcin

### Content of the Invention

The purpose of the present invention is to provide an antibiotic with a novel structure and a unique mechanism for treating diseases and their complications (CDI) caused by *C. difficile,* as well as to provide formulations of thiopeptcin for oral administration and their corresponding technologies of formulation according to CDI and the characteristics of intestinal administration.

The specific technical scheme of the invention is as follows:
The use of thiopeptcin in the preparation of drugs for treating diseases and their complications caused by *C. difficile.*

The aforementioned diseases and their complications caused by *C. difficile* include pseudomembranous colitis, colitis, toxic colitis, diarrhea, pseudomembranous colitis, pyelonephritis, meningitis, abdominal pain, vaginal infections bacteremia, gas gangrene, antibiotic-related diseases and their complications caused by *C. difficile* infection.

According to above applications, thiopeptcin can be used in treating infections caused by *C. difficile,* including sensitive strains and antibiotic-resistant *C. difficile.* The above mentioned antibiotics include ampicillin, cephalosporin, lincomycin, clindamycin, erythromycin, tetracycline, vancomycin and metronidazole.

In the applications according to the present invention, thiopeptcin is administered *via* enema, nasogastric tube, rectal or oral route, preferably orally administered.

Another purpose of the present invention is to provide a type of pharmaceutical preparations for treating diseases and complications caused by *C. difficile,* in which thiopeptcin is an active component in the pharmaceutical preparations.

In the above pharmaceutical preparations, preference is given to formulations of thiopeptcin for oral administration, including tablets, capsules, film-coated tablets, chewable tablets, sugar-coated granules, suspensions, syrup, emulsion, freeze-dried powder for oral administration, liquid for oral administration, mucilage, effervescent, granules or tincture and related formulations with sustained- or controlled-released characteristics.

The above-mentioned formulations of thiopeptcin for oral administration contain thiopeptcin and pharmaceutically acceptable materials, including one or more of adhesives, supporting materials, fillers, lubricants, dispersants, glidants, colorants, emulsifiers, stabilizers, solubilizers, cosolvents, pH adjustors, antioxidants, flavoring agents, preservatives, materials for sustained-release and controlled-release preparations.

In above-mentioned formulations of thiopeptcin for oral administration, the adhesives are selected from one or more of polyvinylpyrrolidone, starch, hypromellose, hydroxypropylcellulose, microcrystalline cellulose, methylcellulose, ethylcellulose, and carboxymethyl, cellulose and its sodium salt, gelatin, gum arabic, guar gum, tragacanth, sodium alginate. Supporting materials are selected from one or more of croscarmellose sodium, cross-linked polyadipone, starch, sodium carboxymethyl starch, carboxypropyl starch, microcrystalline cellulose, low substituted hydroxypropyl cellulose. Fillers are selected from one or more of lactose, sucrose, starch, modified starch, mannitol, sorbitol, dextrin derivatives (such as dextrin, maltodextrin), cellulose derivatives (such as microcrystalline cellulose, cellulose), calcium sulfate. Lubricants are selected from one or more of stearic acid, calcium stearate, magnesium stearate, hydrogenated vegetable oil, wax of palmitic acid, talc, polyethylene glycol, sodium stearyl fumarate, etc. Glidants are selected from micro-silica gel and talc. Dispersants are selected from one or more of microcrystalline cellulose, lactose, mannitol, calcium hydrogen phosphate. Emulsifiers are selected from one or more of SLS, Tween, poloxamers, povidone, lecithin, stearates, gelatin, methylcellulose, hydroxypropylcellulose, polyoxyethylene, castor oil, beeswax, acetyl alcohol, egg yolk phospholipids or soy phospholipids. Stabilizers are selected from one or more of poloxamers, polyethylene glycols, polysorbates. Solubilizers are selected from one or more of polyethylene glycol, fatty acids, sorbitan, polysorbate, povidone, poloxamer, cyclodextrin and lecithin. Cosolvents are selected from one of more of ethanol, propylene glycol, DMSO, glycerol, and polyethylene glycol. pH adjustors are selected from one or more of hydrochloric acid, acetic acid, phosphoric acid, carbonic acid, citric acid, phosphoric acid, lactic acid, tartaric acid, malic acid, succinic acid, sodium hydroxide, triethanolamine, ethylenediamine or the buffer composed of the corresponding acid and its salt. Antioxidants are selected from one or more of sodium metabisulfite, sodium bisulfite, sodium sulfite, sodium thiosulfate, dibutyl phenol and ascorbic acid. Flavoring agents are selected from one or more of saccharine, monosaccharide syrup, juice syrup, disodium glycyrrhizinate, sucrose, sucralose, aspartame, stevia, maltitol, sorbitol, xylitol, lactitol, mannitol, natural flavors and artificial flavors. Preservatives are selected from one or more of benzoic acid, sodium benzoate, parabens, sorbic acid/potassium sorbate. Materials for sustained-release are selected from one or more of PLGA, PLA, chitosan and derivatives, methacrylic acid copolymers, lactic acid-lysine copolymer, hydroxypropyl methylcellulose, gelatin, polyacrylic acid and beeswax. Materials for controlled-release are selected from one or more of high molecular gels, chitosan and its blends, cyclodextrins and derivatives, albumin, polylactic acid and its copolymers, sodium alginate and cellulose.

Unlike common bacterial infectious diseases, the cause of *C. difficile* infection is due to excessive or unreasonable use of broad-spectrum antibiotics, which breaks the balance of intestinal flora, resulting in the accumulation of a large number of *C. difficile* and a large amount of exotoxins A and B. In addition, after an excessive or unreasonable use of broad-spectrum antibiotics, the sensitivity of *C. difficile* to drugs is reduced, even antibiotic-resistant strains have emerged. A large number of clinical studies have found that *C. difficile* is resistant to ampicillin, cephalosporins, lincomycin, clindamycin, erythromycin and tetracycline during the treatment of infections caused by *C. difficile.* Currently available drugs for treating infection caused by *C. difficile* are very limited, among which vancomycin and metronidazole are usually used. However, with the emergence of reduced-sensitivity and drug-resistant strains of these two drugs, the treatment for the infections caused by *C. difficile* is facing great challenges with current treating options, there is no ideal antibacterial drug that can effectively treat infections caused by *C. difficile* at present.

The present invention aims at *C. difficile* ATCC 9689, clinically isolated and drug-resistant strains, and measures the antibacterial activity of thiopeptcin using the broth dilution method recommended by CLSI, as well as using MIC (minimum inhibitory concentration) values as the index of the evaluation of the antibacterial activity of *C. difficile* and its drug-resistant strains. The results show that the bactericidal activity of thiopeptcin to *C. difficile* and its drug-resistant strains is significantly better than those of vancomycin and metronidazole.

The pharmaceutical formulations of thiopeptcin for oral administration according to the present invention can be prepared by conventional techniques in this field.

The therapeutic dosage of thiopeptcin in the present invention is 0.001mg/kg-10,000 mg/kg per day, preferably 0.001-1,000 mg/kg per day. The unit dose of formulations of thiopeptcin for oral administration is from 0.001 to 10,000 mg, preferably from 0.01 to 100 mg.

Given that *C. difficile* exists in human intestines, the present invention selects the formulations for oral administration as a treatment of the infections caused by *C. difficile* as well as investigates oral bioavailability of thiopeptcin. The results show that no thiopeptcin enters the blood circulation after intragastric administration in rats, and the plasma concentration of thiopeptcin is below quantitative detection limit (1 ng/mL), indicating that thiopeptcin does not enter human blood circulation and has a direct effect on the intestinal tract, resulting in none or infinitesimal potential for side effects.

Compared to existing CDI therapeutics and treatment methods, the present invention has following advantages.
1. Thiopeptcin, a thiopeptide antibiotic, has a unique structure, a completely new mechanism and strong antibacterial activity. The results of the present invention show that thiopeptcin exhibits superior antibacterial activity against *C. difficile* and its drug-resistant strains.
2. The antibacterial activity (MIC value) of thiopeptcin to drug-sensitive and drug-resistant *C. difficile* strains is superior to that of the antibiotics for clinical treatment of CDI, such as vancomycin.
3. After oral administration, thiopeptcin is not absorbed in the intestine and does not enter blood circulation, indicating that it exhibits better safety profiles without any systemic side effects. In addition, the variety of thiopeptcin formulations for oral administration is convenient for patients to choose an appropriate type and route of administration.

In summary, thiopeptcin possesses strong activity of inhibiting *C. difficile* and can be used in the development of formulations for oral administration for treating CDI with a significant value for clinical application.

### Specific Methods

The following examples are used for understanding the present invention and are only for illustrative purposes without limiting the scope of application of the invention.

### Example 1 In vitro antibacterial activity of thiopeptcin against C. difficile and its drug-resistant strains

Thiopeptidin is tested for its antibacterial activity *in vitro* against strain sensitive to *C. difficile,* such as *C. difficile* ATCC 9689, clinically isolated *C. difficile* LCN 001, and clinically isolated resistant strains, the results are compared with those of vancomycin. The minimum inhibitory concentration (MIC) of thiopeptcin is determined according to the broth dilution method recommended by CLSI M11-A7. According to the method, the Brucella broth supplemented with Hemin (5 mg/ml), vitamin K1 (1 µg/ml), lysed horse blood (5%) and oxidase (1:25 v/v) was divided into different assay groups, and then they are added with either thiopeptcin or vancomycin in DMSO. The concentrations of tested drugs range from 0.025 µg/ml to 128 µg/ml. The growth of *C. difficile* in every type of culture medium was examined, and the minimum antibacterial drug concentration at which the bacteria do not grow was the desired MIC. As shown in Table 1, the antibacterial activity of thiopeptcin against *C. difficile* and its drug-resistant strains was significantly better than that of vancomycin.

**Table 1 Sensitivity of C. difficile to Thiopeptcin (MIC)**

| Strain | Thiopeptcin(µg/ml) | Vancomycin(µg/ml) |
|---|---|---|
| *C. difficile* ATCC 9689 | 0.005 | 0.25 |
| clinically isolated strain LCN 001 | 0.025 | 0.5 |
| Reduced-sensitivity strain to Vancomycin *C. difficile* LCN 0012 | 0.025∼0.05 | 4∼8 |
| Reduced-sensitivity strain to Metronidazole *C. difficile* LCN 0048 | 0.005∼0.01 | 0.8∼3 |
| Clindamycin-resistant strain *C. difficile* LCN 0027 | 0.025 | 1.5 |
| Erythrocin-resistant strain *C. difficile* LCN 0081 | 0.005 | 1.5 |

### Example 2 LC-MS/MS detection of thiopeptcin

30 µl of blood or urine sample was added into 90 µl of methanol containing 5 ng/ml verapamil. After vortexing for 3 min and centrifuging at 14,000 rpm for 5 min, 80 µl of supernatant was taken for LC-MS/MS analysis.

Conditions for LC were as follows:
Column: Waters Symmetry 300 C18 3.5 µm 2.1* 100 mm;
Mobile phase A: 10 mM ammonium acetate + 0.02% aqueous ammonia solution;
mobile phase B: methanol;
Duration of analysis: 5.2 min; injection volume: 5 µL; column temperature: 25°C;
gradient elution:

| Time (min) | Flow rate (mL/min) | B% |
|---|---|---|
| 0.00 | 0.4 | 67 |
| 2.40 | 0.4 | 67 |
| 2.45 | 0.4 | 90 |
| 3.80 | 0.4 | 90 |
| 3.85 | 0.4 | 67 |
| 5.20 | 0.4 | 67 |

Conditions for MS/MS analysis were as follows:
Mass Spectrometer: Positive Ion Mode (Agilent 6460B)
Detection of ion pairs: Thiopeptcin: 1437.3-172.0, fragment = 135, CE = 22;
Verapamil (internal standard): 455.2→165.1, fragment=135, CE=25;
Mass spectrometry parameters: gas temp at 350 °C; gas flow at 10 L/min; nebulizer of 40 psi; sheath gas flow at 10 L/min and sheath gas heater at 400 °C.

### Example 3 Pharmacokinetics of Thiopeptcin after oral administration

Preparation of thiopeptcin: Certain amount of thiopeptcin was dissolved in a mixed solvent of pH 5.0 containing 10% PEG400, 1% Tween 80 and 5 mg/ml citric acid. The solution was shaken to be clear for intragastric administration.

Male rats were divided into 4 groups with high, medium, and low doses according to experimental purposes. The high dose group was 50 mg/kg, the middle dose group was 25 mg/kg, the low-1 dose group was 5 mg/kg and the low-2 dose group was 1 mg/kg. Approximately 0.3 ml of blood samples were collected at approximately 15 min, 30 min, 45 min, 1, 2, 4, 6, 8 and 24 h after administration for bioavailability determination. See Example 2 for the method of analysis.

**Table 2 Plasma concentrations of thiopeptcin after orally administrated in rats (ng/ml)**

| Time/h | ♂ | | | |
|---|---|---|---|---|
| | High | Middle | Low 1 | Low 2 |
| 0.25 | N.D. | N.D. | N.D. | N.D. |
| 0.50 | N.D. | N.D. | N.D. | N.D. |
| 0.75 | N.D. | N.D. | N.D. | N.D. |
| 1 | N.D. | N.D. | N.D. | N.D. |
| 2 | N.D. | N.D. | N.D. | N.D. |
| 4 | N.D. | N.D. | N.D. | N.D. |
| 6 | N.D. | N.D. | N.D. | N.D. |
| 8 | N.D. | N.D. | N.D. | N.D. |
| 24 | N.D. | N.D. | N.D. | N.D. |

| | | | | |
|---|---|---|---|---|
| N.D. represents not detectable (below detection limit) | | | | |

From Table 2, after intragastric administration of thiopeptcin in rats, no thiopeptcin entered blood circulation, and the plasma concentration was lower than the lower limit of quantification (1 ng/mL), indicating that thiopeptcin is not absorbed by the intestinal tract, which in turn exhibits better safety profiles without any systemic side effects.

### Example 4 Thiopeptcin Tablets

The materials in the formulation of thiopeptcin tablets for oral administration are all commonly used and can be used in the pharmaceutical formulation. The examples listed in this invention are only for illustrative purposes and do not limit the scope of application of the invention. The formulation of the example is as follows:

**Regular tablet**

| Ingredient | Quantity (g/1000 tablets) |
|---|---|
| Thiopeptcin | 0.01-250 g |
| Corn starch | 60 g |
| talcum powder | 25 g |
| PEG 400 | 2 g |
| Magnesium stearate | 3 g |

**Preparation:** Thiopeptcin and corn starch were mixed and passed through 100 mesh sieves. Under continuous stirring, the binder solution was prepared by heating water with PEG400 at 45°C. Binder solution was sprayed onto the mixture of thiopeptcin and starch and dried in a 60°C input air, finally talc and magnesium stearate were added. The above powder was taken for tableting to obtain thiopeptcin tablets.

**Dispersible tablets**

| Ingredient | Quantity (g/1,000 tablets) |
|---|---|
| Thiopeptcin | 0.01-250 g |
| Lactose | 30 g |
| Microcrystalline cellulose | 90 g |
| Low-substituted hydroxypropyl cellulose | 25 g |
| Tween 80 | 5.0 g |
| Micro silica gel | 15 g |
| Magnesium stearate | 9 g |
| Aspartame | 2 g |

**Preparation:** Thiopeptcin and micro-silica gel were mixed and passed through 100 mesh sieves. Meanwhile, lactose, microcrystalline cellulose, low-substituted hydroxypropyl cellulose and aspartame were mixed. The two types of powder were passed through 80 mesh sieves and mixed evenly. Tween 80 and magnesium stearate were added to a mixing machine and mixed for more than 10 minutes. The pressure and weight of the tableting machine were adjusted, and the above powder was taken for tableting to acquire thiopeptcin dispersible tablets.

**Tablets for sustained-release**

| | Ingredient | Quantity (g/ 1,000 tablets) |
|---|---|---|
| Thiopeptcin tablet | Thiopeptcin | 0.01-250 g |
| | Hydroxypropyl methyl cellulose | 85 g |
| | Polyvinylpyrrolidone | 9 g |
| | Magnesium stearate | 5 g |
| | Talcum powder | 2.5 g |
| | Lactose | 87.5 g |
| | Citric acid | 50 g |
| Sustained-release coat | Hydroxypropyl methyl cellulose | 5 g |
| | Propylene glycol | 2 g |
| | Castor oil | 2 g |
| | Tween 80 | 2 g |
| | talcum powder | 4.5 g |
| | Lemon yellow | 0.2 g |
| | Food essence | 1.2 g |

### Preparation:

1) Polyvinylpyrrolidone was dissolved in an ethanol solution and stirred into a uniform solution. After mixing 80-100 mesh thiopeptcin, hydroxypropyl methylcellulose, citric acid and lactose uniformly, polyvinylpyrrolidone alcohol solution was added to obtain a soft material. After the soft material was dried, magnesium stearate and talc powder were added, and the mixture was evenly mixed and tableted to obtain thiopeptcin tablets.
2) The talc powder was sieved and added to an alcohol solution containing hydroxypropylmethyl cellulose, castor oil, propylene glycol and Tween-80, and the aqueous solution of lemon yellow and edible essence was added after stirring to prepare slow release coating solution.
3) Thiopeptcin tablets were added to the coating pan and the amount of the intake air was adjusted. After pre-warm of the tablets, the temperature for coating was maintained at 30-50 °C and then sustained-release tablets for thiopeptcin were acquired after drying.

Controlled-release tablets (enteric coated tablets)

| | Ingredient | Quantity (g/ 1,000 tablets) |
|---|---|---|
| Thiopeptcin tablet | Thiopeptcin | 0.01-250 g |
| | Hydroxypropylmethylcellulose | 85 g |
| | Polyvinylpyrrolidone | 9 g |
| | Magnesium stearate | 5 g |
| | Talcum powder | 2.5 g |
| | Lactose | 87.5 g |
| | Citric acid | 50 g |
| Enteric coat | Poly Acrylic Resin 11 | 10 g |
| | Ethanol | 130 g |
| | Diethyl phthalate | 2 g |
| | Castor oil | 4 g |
| | Tween 80 | 2.2 g |
| Sugar coat | Sucrose | 58 g |
| | talcum powder | 58 g |
| | Gelatin | 20 mg |
| | Kawasaki | 280 mg |
| | Dimethicone | 28 mg |

### Preparation:

1) Preparation of thiopeptcin tablet is the same as sustained-release tablet.
2) Poly-acrylic resin 11 was added into ethanol and stirred until dissolved, then diethyl phthalate, Tween 80 and castor oil were added, stirred well and sieved for use.
3) Using purified water to dissolve sucrose into syrup, filtered it while hot.
4) Thiopeptcin tablets were added to the coating pan, and the amount of the air intake was adjusted. After pre-warm of tablets, the temperature was controlled at 30 -50 °C, syrup was added to make the surface of the core evenly moist, and then talc powder was added to wrap the surface of the core and dry them for use. The above procedure was then repeated to coat them evenly to obtain thiopeptcin enteric controlled-release tablets.

### Example 5 Thiopeptcin chewable tablets

The materials in the formulation of thiopeptcin chewable tablet for oral administration are commonly used and can be used in the pharmaceutical formulation. The examples listed in this invention are for illustrative purposes only and do not limit the scope of application of the invention. The formulation of the example is as follows:

**Chewable tablets**

| Ingredient | Quantity (g/ 1,000 tablets) |
|---|---|
| Thiopeptcin | 0.01-250 g |
| Lactose | 200 g |
| Sodium cyclamate | 50 g |
| Aspartame | 20 g |
| Magnesium stearate | 50 g |
| Fresh milk flavor | 100 g |
| Ethanol | 20 g |

**Preparation:** Thiopeptcin and excipients were sieved through 100 mesh sieves. Thiopeptcin, lactose, cyclamate and aspartame were mixed and added with ethanol to make a soft material, and then passed through 20-50 mesh sieves, dried under vacuum at 50°C, then sifted through 20 mesh sieves again. Finally magnesium stearate and fresh milk flavor were added and mixed well. The above powder was taken for tableting to obtain thiopeptcin chewable tablets.

### Example 6 Thiopeptcin capsules

The materials in the formulation of thiopeptcin capsules are all commonly used and can be used in the pharmaceutical formulation field. The examples listed in this invention are for illustrative purposes only and do not limit the scope of application of the invention. The formulation of the example is as follows:

**Regular capsules**

| Ingredient | Quantity (g/ 1,000 capsules) |
|---|---|
| Thiopeptcin | 0.01-250 g |
| Pregelatinized starch | 200 g |
| Polyvinylpyrrolidone aqueous solution (8%) | 15 g |
| talcum powder | 75 g |
| Magnesium stearate | 37.5 g |

**Preparation:** Thiopeptcin and the materials were crushed and passed through 100 mesh sieves for use. Thiopeptcin was mixed well with pregelatinized starch, and then polyvinylpyrrolidone aqueous solution (8%) was added as a binder to prepare a soft material. Talc powder and magnesium stearate were added, and pellets were prepared by extrusion rounding method to control the moisture of the pellets to be 40% or less. The acquired pellets were filled into capsule shells to obtain thiopeptcin capsules.

**Soft capsules**

| Ingredient | Percentage or Quantity (g/ 1,000 capsules) |
|---|---|
| Thiopeptcin | 0.01-250 g |
| Gelatin | 46% |
| PEG-400 | 0.1 L |
| Glycerin | 17.8% |
| Tween-80 | 0.02 L |
| Water | 36.2% |
| Povidone K30 | 30 g |

**Preparation:** Povidone was dissolved in PEG-400 and Tween-80, and stirred well. After the addition of thiopeptcin, it was stirred to dissolve. Then, the solution was made into a capsule to acquire thiopeptcin soft capsules.

**Capsules for sustained-release**

| Drug loaded pellets | |
|---|---|
| Ingredient | Quantity (g/ 1,000 capsules) |
| Thiopeptcin | 0.01-250 g |
| Microcrystalline cellulose | 70 g |
| Sugar powder | 40 g |
| 5% hydroxypropyl cellulose | Proper amount |
| Sodium dialkylsulfate | 0.3 g |
| Coating solution (For 500g drug-loaded pellets) | |
| Ingredient | Percentage or Quantity(g/ 1,000 capsules) |
| Methacrylic acid copolymer | 27% |
| talcum powder | 28% |
| Polyethylene glycol 6000 or ethanol | 1 g |
| Sodium dodecyl sulfate | 0.5 g |
| Water | Proper amount to 100% |

### Preparation:

1) Preparation of blank Pellet Cores: Commercially available blank pellets can be used or prepared;
2) Preparation of capsules for sustained-release: methacrylic acid copolymer, talc, sodium dialkylsulfate and polyethylene glycol were mixed as a coating solution. 500 g 20 mesh drug-containing pellets were placed in a centrifugal coating granulator for coating. After filling capsules with above drug-containing coated pellets, thiopeptin capsules for sustained-release were obtained.

### Example 7 Sugar coated pills

The materials in the formulation of thiopeptcin sugar coated pills are all commonly used and can be used in the pharmaceutical formulation. The examples listed in this invention are for illustrative purposes only and do not limit the scope of application of the invention. The formulation of the example is as follows:

**Sugar coated pills**

| Ingredient | Quantity (g/ 1,000 pills) |
|---|---|
| Thiopeptcin | 10 g |
| Microcrystalline cellulose | 25 g |
| Carboxymethyl starch sodium | 6 g |
| Lactose | 1.5 g |
| Ethanol (95%) | 85 g |
| Polyacrylic resin | 4.4 g |
| Polyethylene glycol 6000 | 0.9 g |
| Magnesium stearate | 1.5 g |
| Sucrose | 25 g |
| Essence | Proper amount |
| Water | Proper amount to 100% |

**Preparation:** 80-mesh thiopeptcin, microcrystalline cellulose, carboxymethyl starch sodium and lactose were mixed in a highly efficient wet machine, and an appropriate amount of water was added and stirred to give a soft material, and an orifice with 0.8 mm pore size is used to extrude it. After spheronnizing it in a spheronizer with a speed of 800-2,000 rpm, it was dried at 50-55 °C to obtain thiopeptcin pills.

After adding polyacrylic acid resin, polyethylene glycol 6000, flavor and sucrose into ethanol in container while stirring, an appropriate amount of water was added and mixed well, then magnesium stearate was added, stirred and suspended the coating solution for use. Then, thiopeptcin was placed in a coating pan, and the temperature of inlet air was adjusted to 40-60 °C. Then, the atomization pressure and the liquid supplement speed were adjusted, and the coating liquid was uniformly wrapped on the surface of the pellets. The thiopeptcin sugar coated pills were obtained by drying for about 30 min after the coating is completed.

### Example 8 Thiopeptcin dry suspension

The materials in the formulation of thiopeptcin dry suspension are all commonly used and can be used in the pharmaceutical formulation. The examples listed in this invention are for illustrative purposes only and do not limit the scope of application of the invention. The formulation of the example is as follows:

**Regular dry suspension**

| Ingredient | Quantity (g) |
|---|---|
| Thiopeptcin | 0.01-250 g |
| Mannitol | 20 g |
| Sodium cyclamate | 40 g |
| Hypromellose | 30 g |
| Ethanol (50%) | 2 g |

**Preparation:** 120-mesh thiopeptcin, mannitol, cyclamate and hydroxypropyl methyl cellulose were mixed evenly before passing through 120 mesh sieves, then ethanol was added and mixed well to prepare a soft material. After passing it through 30 mesh sieves, thiopeptcin dry suspension was obtained after drying and packing.

**Dry suspension for sustained-release**

| Ingredient | Quantity (g) |
|---|---|
| Thiopeptcin | 0.01-250 g |
| Blank pellets | 0.1 g |
| Povidone | 1.8 g |
| Hypromellose | 1 g |
| Tween-80 | 0.08 g |
| Polyethylene glycol-4000 | 0.1 g |
| Ethyl cellulose | 0.6 g |
| Sodium phosphate | 0.3 g |
| Sucrose | 2 g |
| Sodium carboxymethyl cellulose | 0.06 g |

**Preparation:** After mixing 120-mesh thiopeptcin with povidone, 95% ethanol was added and stirred to obtain a suspension. The blank pellets were placed in a fluidized bed and sprayed with thiopeptcin solution and dried to prepare thiopeptcin-loaded pellets. After adding Tween-80 and hypromellose to 75% ethanol, they were stirred till completely dissolving to obtain a coating solution, and the coating solution was sprayed into a fluidized bed to prepare thiopeptcin-coated pellets. Ethylcellulose, povidone and polyethylene glycol-4000 were completely dissolved in 95% ethanol under stirring to prepare a sustained-release layer coating solution and sprayed into a fluidized bed to prepare thiopeptcin pellets for sustained-release. Finally, the drug-loaded sustained-release pellets were mixed and packed with sodium phosphate, sucrose and sodium carboxymethyl cellulose to form a thiopeptcin dry suspension for sustained-release.

### Example 9 Thiopeptcin syrup

The materials in the formulation of thiopeptcin syrup are all commonly used and can be used in the pharmaceutical formulation field. The example listed in this invention is for illustrative purposes only and does not limit the scope of application of the invention. The formulation of the example is as follows:

**Syrup**

| Ingredient | Quantity (g) |
|---|---|
| Thiopeptcin | 0.01-250 g |
| Glutamate | 8 g |
| β-Cyclodextrin | 35 g |
| Thiourea | 1 g |
| EDTA-2Na | 0.3 g |
| Benzoic acid | 2 g |
| Hydroxyphenethyl ester | 0.5 g |
| Strawberry flavor | 1 g |
| Food coloring | 0.005 g |
| Sucrose | 650 g |
| Water | 1000 ml |

**Preparation:** Sucrose was added to 300 ml purified water, stirred and heated until boiling, making sure it is completely dissolved to form syrup, which was cooled and filtered for use (solution A). Food coloring was added to 1 ml water and stirred until dissolving (solution B). After 230 ml purified water was heated and boiled, cyclohexene cyclodextrin, thiourea, EDTA-2Na, benzoic acid and ethyl hydroxybenzoate were added and stirred until completely dissolving. Thiopeptcin and thiourea were added when the temperature was cooled below 60 °C and stirred until dissolving (solution C). Then, solution B was added to solution C, stirred well and slowly added to solution A through 300 mesh sieves while stirring evenly. When the temperature droped below 40 °C, strawberry essence was added and mixed well. The remaining purified water was added and filtered the mixture through 300 mesh sieves to obtain thiopeptcin syrup.

### Example 10 Thiopeptcin Emulsion for Oral Administration

The materials in the formulation of thiopeptcin emulsion for oral administration are commonly used and can be used in the pharmaceutical formulation. The examples listed in this invention are for illustrative purposes only and do not limit the scope of application of the invention. The formulation of the example is as follows:

**Emulsion for oral administration**

| Ingredient | Quantity (g/1,000 ml) |
|---|---|
| Thiopeptcin | 0.01-250 g |
| Soybean oil | 200 ml |
| Lecithin | 10 g |
| Poloxam | 20 g |
| 0.9% saline | 800 ml |
| Saccharin | 2 g |
| Orange Flavor | 2 g |

**Preparation:** Poloxamer was dispersed and ground in 0.9% sodium chloride solution evenly, then oil phase containing dissolved thiopeptcin and lecithin was added and stirred hard into premulsion. After adding saccharin and essence, the premulsion was diluted and quantified with 0.9% saline solution to obtain a uniformly dispersed emulsion.

### Example 11 Freeze-dried powder of thiopeptcin for oral administration

The materials in the formulation of thiopeptcin freeze-dried powder for oral administration are commonly used and can be used in the pharmaceutical formulation. The example listed in this invention is for illustrative purposes only and does not limit the scope of application of the invention. The formulation of the example is as follows:

**Freeze-dried powder for oral administration**

| Ingredient | Percentage or Quantity (g) |
|---|---|
| Thiopeptcin | 0.01-250 g |
| PEG-400 | 10% |
| Dextran | 5% |
| Citric acid | 0.5% |
| Saccharin | 1% |

**Preparation:** Thiopeptcin was mixed with PEG-400, stired until dissolved (solution A); citric acid, dextran and saccharin were dissolved with purified water and stirred until completely dissolving (solution B); solution A and B were mixed and pH was adjusted to 5.0 with 0.5 M citric acid or 0.5 N sodium hydroxide solution; water was added to dilute and quantify. Finally, the solution was freeze-dried to obtain freeze-dried powder of thiopeptcin for oral administration.

### Example 12 Thiopeptcin mucilage

The materials in the formulation of thiopeptcin mucilage are commonly used and can be used in the pharmaceutical formulation. The example listed in this invention is for illustrative purposes only and does not limit the scope of application of the invention. The formulation of the example is as follows:

**Mucilage**

| Ingredient | Quantity (g) |
|---|---|
| Thiopeptcin | 0.01-250 g |
| Glycerol | 40 g |
| Propylene glycol | 10 g |
| Polyoxyethylene copolymer | 10 g |
| Polyethylene oxide-8000 | 30 g |
| Chlorhexidine Acetate | 0.7 g |
| EDTA-2Na | 0.1 g |
| Water | Proper amount |

**Preparation:** Thiopeptcin (100-mesh) was suspended in purified water, glycerol, propylene glycol, polyoxyethylene copolymer and polyethylene oxide-8000 were added. The mixture was stirred until the materials were dissolved to form a colloidal solution (solution A). Chlorhexidine acetate and EDTA-2Na were dissolved in 200 ml water and added to solution A. After mixing well and adjusting pH to 5-6, thiopeptcin mucilage was obtained.

### Example 13 Effervescent granules or tablets of thiopeptcin

The materials in the formulation of effervescent granules or tablets of thiopeptcin are commonly used and can be used in the pharmaceutical formulation. The examples listed in this invention are for illustrative purposes only and do not limit the scope of application of the invention. The formulation of the example is as follows:

**Effervescent Granules or Tablets**

| Ingredient | Percentage or Quantity (g) |
|---|---|
| Thiopeptcin | 0.01-250 g |
| Sodium bicarbonate | 4.3% |
| Sodium carbonate | 5.8% |
| Citric acid | 8.5% |
| Fumaric acid | 1.8% |
| Polyethylene glycol | 15% |
| Sodium dialkylbenzene sulfonate | 6% |
| Sodium carboxymethyl cellulose | 6% |
| Azone | 6% |
| diatomite | Proper amount |

**Preparation:** The amount of each material according to the above ratio was calculated and weighed. Thiopeptcin, diatomaceous earth, carboxymethylCellulose sodium, citric acid and fumaric acid were mixed evenly, and then azone was added and stirred. After being mixed evenly, all other remaining materials were finally added. After being pulverized one time by a micropulverizer, they were passed through 200 mesh sieves and loaded into the granulator (or tablet press). After one-time extrusion to form the shape of granule (or tablet compression) and drying, thiopeptcin granules (or tablets) were obtained.

### Example 14 Thiopeptcin Granules

The materials in the formulation of thiopeptcin granules are commonly used and can be used in the pharmaceutical formulation. The example listed in this invention is for illustrative purposes only and does not limit the scope of application of the invention. The formulation of the example is as follows:

**Granules**

| Ingredient | Quantity (g) |
|---|---|
| Thiopeptcin | 1 g |
| Sucrose | 20 g |
| Pregelatinized starch | 2 g |
| Sodium saccharin | 0.1 g |
| Sodium carboxymethyl cellulose | 0.1 g |
| Povidone (4%) | 15 ml |
| Sweet Orange Flavor | 0.1 g |

**Preparation:** Sucrose, pregelatinized starch, sodium saccharin, sodium carboxymethyl cellulose were mixed in a wet mixing granulator, and then mixed with a solution of povidone (4%). 80-100 mesh thiopeptcin and sweet orange flavors were added in the granulator to acquire thiopeptcin granules for oral administration.

### Example 15 Thiopeptcin solution for oral administration

The materials in the formulation of thiopeptcin solution for oral administration are commonly used and can be used in the pharmaceutical formulation. The example listed in this invention is for illustrative purposes only and does not limit the scope of application of the invention. The formulation of the example is as follows:

**Solution for oral administration**

| Ingredient | Quantity (g/1000 ml) |
|---|---|
| Thiopeptcin | 0.01-250 g |
| Sodium dihydrogen phosphate | 30 g |
| Sodium benzoate | 3 g |
| Sodium saccharin | 0.1 g |
| Tannic acid | 1.5 g |
| Sucrose | 350 g |
| Water | Remaining amount |

**Preparation:** Thiopeptcin, cosolvents and preservatives were dissolved in 100 ml purified water and stirred until completely dissolving (solution A). The flavoring agent was dissolved in 500 ml water, and then the solution was added to solution A and additional pure water was added to 1000ml. The mixture was mixed and filtered to obtain thiopeptcin solution for oral administration.

### Example 16 Thiopeptcin tincture

The materials in the formulation of thiopeptcin tincture are commonly used and can be used in the pharmaceutical formulation. The example listed in this invention is for illustrative purposes only and does not limit the scope of application of the invention. The formulation of the example is as follows:

**Tincture**

| Ingredient | Quantity (g) |
|---|---|
| Thiopeptcin | 0.01-250 g |
| 30-70% white wine | 4 to 20 times the weight of thiopeptcin |
| Fatty acid sorbitan | 1% the amount of white wine |
| Food essence | 0.1% the amount of white wine |

**Preparation:** Thiopeptcin, fatty acid sorbitan and edible flavor were dissolved in white wine, mixed and filtered to obtain thiopeptcin tincture.

## Claims

1. The use of thiopeptcin in the preparation of pharmaceutical agents for treating infectious diseases and their complications caused by *C. difficile.*

2. The use of thiopeptcin according to claim 1, wherein the infectious diseases and their complications include pseudomembranous colitis, colitis, toxic colitis, diarrhea, pseudomembranous colitis, and pyelonephritis, meningitis, celiac and vaginal infections, bacteremia and gas gangrene, antibiotic-related diseases and their complications caused by *C. difficile* infections.

3. The use of thiopeptcin according to claim 1 and 2, wherein the *C. difficile* includes sensitivity-reduced or antibiotic-resistant strains.

4. The use of thiopeptcin according to claim 3, wherein the antibiotic includes one or more of ampicillin, cephalosporin, lincomycin, clindamycin, erythromycin, tetracycline, vancomycin and metronidazole.

5. The use of thiopeptcin according to claim 1, thiopeptcin is administered via enema, nasogastric, rectal or oral routes.

6. A pharmaceutical formulation for treating infectious diseases and complications caused by *C*. *difficult,* wherein the active component of the pharmaceutical formulation is thiopeptcin.

7. The pharmaceutical formulation according to claim 6, wherein the pharmaceutical formulation is an oral formulation, and wherein the oral formulation includes tablets, capsules, film-coated tablets, chewable tablets, sugar-coated granules, suspension, syrup, emulsion, freeze-dried powder for oral administration, liquid for oral administration, mucilage, effervescent, granules, or tincture and their related sustained-release formulations.

8. The pharmaceutical formulation according to claim 7, wherein the pharmaceutical formulation are consisted of thiopeptcin and pharmaceutically usable materials including one or more of adhesives, supporting materials, fillers, lubricants, dispersants, glidants, colorants, emulsifiers, stabilizers, solubilizers, latent solvents, pH adjusters, antioxidants, flavoring agents, preservatives, materials for sustained-release and materials for controlled-release.

9. The pharmaceutical formulation according to claim 8, wherein the adhesives are selected one or more of polyvinylpyrrolidone, starch, hypromellose, hydroxypropyl cellulose, microcrystalline cellulose, methyl cellulose, ethyl cellulose, carboxymethyl cellulose and its sodium salt, gelatin, gum arabic, guar Rubber, tragacanth and sodium alginate. Supporting materials are selected from one or more of croscarmellose sodium, polyvinylpolypyrrolidone, starch, sodium carboxymethyl starch, carboxypropyl starch, microcrystalline cellulose, low substituted hydroxypropyl cellulose. Fillers are selected from one or more of lactose, sucrose, starch, modified starch, mannitol, sorbitol, dextrin derivatives, cellulose derivatives and calcium sulfate. Lubricants are selected from one or more of stearic acid, calcium stearate, magnesium stearate, hydrogenated vegetable oil, wax of palmitic acid, talc, polyethylene glycol, sodium stearyl fumarate. Glidants are selected from one or more of micro-silica gel and talc. Dispersants are selected from one or more of microcrystalline cellulose, lactose, mannitol and calcium hydrogen phosphate. Emulsifiers are selected from one or more of SLS, Tween, poloxamers, povidone, lecithin, stearates, gelatin, methylcellulose, hydroxypropylcellulose, polyoxyethylene, castor oil, beeswax, cetyl alcohol, egg yolk phospholipids and soy phospholipids. Stabilizers are selected from one or more of poloxamers, polyethylene glycols, polysorbates. Solubilizers are selected from one or more of polyethylene glycol, fatty acids, sorbitan, polysorbate, povidone, poloxamer, cyclodextrin and lecithin. Cosolvents are selected from one of more of ethanol, propylene glycol, DMSO, glycerol and polyethylene glycol. pH adjustors are selected from one or more of hydrochloric acid, acetic acid, phosphoric acid, carbonic acid, citric acid, phosphoric acid, lactic acid, tartaric acid, malic acid, succinic acid, sodium hydroxide, triethanolamine, ethylenediamine or the buffer composed of the corresponding acid and its salt. Antioxidants are selected from one or more of sodium metabisulfite, sodium bisulfite, sodium sulfite, sodium thiosulfate, dibutyl phenol, and ascorbic acid. Flavoring agents are selected from one or more of saccharine, monosaccharide syrup, juice syrup, disodium glycyrrhizinate, sucrose, sucralose, aspartame, stevia, maltitol, sorbitol, xylitol, lactitol, mannitol, natural flavors and artificial flavors. Preservatives are selected from one or more of benzoic acid, sodium benzoate, parabens, sorbic acid/potassium sorbate. Materials for sustained release are selected from one or more of PLGA, PLA, chitosan, methacrylic acid copolymers, lactic acid-lysine polymerization, hydroxypropyl methylcellulose, gelatin, polyacrylic acid, beeswax. Materials for controlled release are selected from one or more of high molecular weight gels, chitosan and its blends, cyclodextrins and derivatives, albumin, polylactic acid and its copolymers, sodium alginate, cellulose.
